# EUROPEAN PATENT APPLICATION

(11) **EP 1 634 912 A1**
(43) Date of publication of application: **15.03.2006**
(21) Application number: 04729247.9
(22) Date of filing: 23.04.2004
(51) Int. Cl.: C08J 9/42, A61L 17/10

(54) **STRETCHED POLYTETRAFLUOROETHYLENE FORMED ARTICLE, METHOD FOR PRODUCTION THEREOF, AND COMPOSITE ARTICLE**

(30) Priority: 13.06.2003 JP 2003168894
(71) Applicant: Sumitomo Electric Industries, Ltd., Osaka-shi, Osaka 541-0041 (JP)
(72) Inventor: HAYASHI, Fumihiro, Sumitomo Electric Ind., Ltd., Osaka-shi, Osaka 5540024 (JP); OKUDA, Yasuhiro, Sumitomo Electric Ind., Ltd., Osaka-shi, Osaka 5540024 (JP)
(74) Representative: Cross, Rupert Edward Blount
(86) International application number: PCT/JP2004/005962
(87) International publication number: WO 2004/111117

(57) **Abstract**

An expanded polytetrafluoroethylene formed article in which a synthetic resin having a tensile elastic modulus higher than the tensile elastic modulus of polytetrafluoroethylene is applied on or impregnated into at least a part of a porous expanded polytetrafluoroethylene rod-shaped article, and the diameter of the part to which the synthetic resin has been applied or impregnated is reduced by applying twist deformation. A composite article including the expanded polytetrafluoroethylene formed article, as well as a method of producing the formed article, is also provided.

## Description

### Technical Field

The present invention relates to a formed article composed of expanded polytetrafluoroethylene and a method of producing the same, and more specifically, to a porous expanded polytetrafluoroethylene rod-shaped article in which at least a part thereof has a reduced diameter with high strength, the porous expanded polytetrafluoroethylene having a porous structure consisting of fibrils and nodes connected by fibrils, and to a method of producing the same. The formed article composed of expanded polytetrafluoroethylene of the present invention is suitable for medical sutures. The suture of the present invention can be used tightly bonded with a surgical needle.

### Background Art

When polytetrafluoroethylene (hereinafter abbreviated to PTFE) is expanded, a porous expanded PTFE is produced. The porous expanded PTFE has a micro fibrous structure including a large number of fibrils (micro fibers) and a large number of nodes (micro nodes) connected by fibrils. This micro fibrous structure forms a porous structure. A rod-shaped article, in particular, a monofilament, composed of a porous expanded PTFE is suitable for a suture because of the following properties: it has low reactivity with living body tissues and a satisfactory sliding property; it is soft; its knot is not easily loosened; it has good extraction characteristic without a scar remaining after the sutures are removed; and it can withstand strong disinfecting conditions.

A rod-shaped article, in particular, a monofilament, composed of a porous expanded PTFE is suitable for a suture because of the following properties: it has low reactivity with living body tissues and a satisfactory sliding property; it is soft; its knot is not easily loosened; it has good extraction characteristic without a scar remaining after the sutures are removed; and it can withstand strong disinfecting conditions.

It is desired that a monofilament suture composed of a porous expanded PTFE has a diameter at least a part of which is reduced so as to increase the tensile strength, to improve the property for bonding with an eye of a needle, and to improve the characteristics for a suture by controlling the porosity, etc.

When living body tissues are bonded using a surgical needle and a suture, preferably, the diameter of the dull edge of the surgical needle (i.e., edge of the surgical needle that is not sharp) is to the extent possible the same as that of the suture so that holes in the tissues formed by penetrating the needle through the living body tissues are filled with the suture.

When the diameter of the dull edge of the surgical needle is excessively larger than the diameter of the suture, the holes in the tissues formed by penetrating the needle cannot be filled with the suture, resulting in the exudation or the leakage of blood.

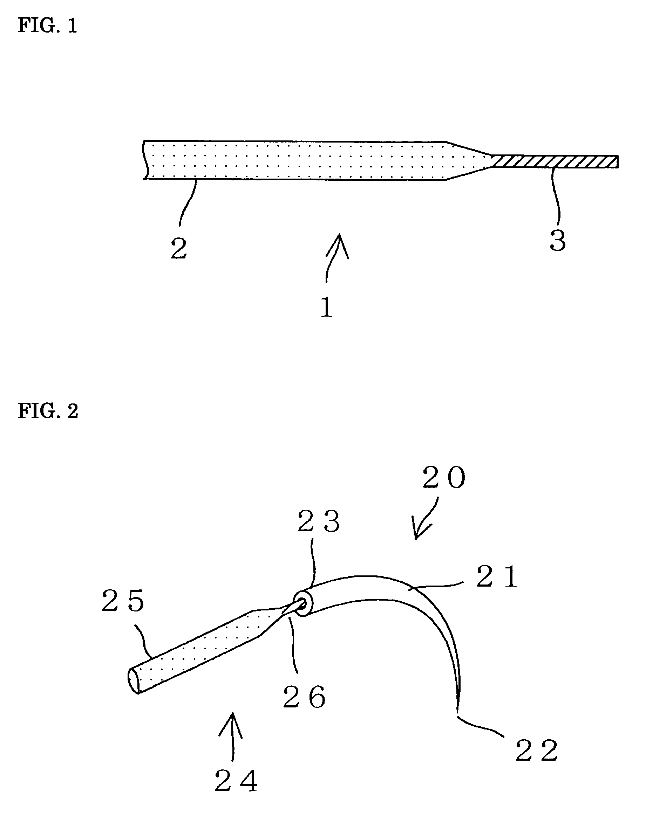

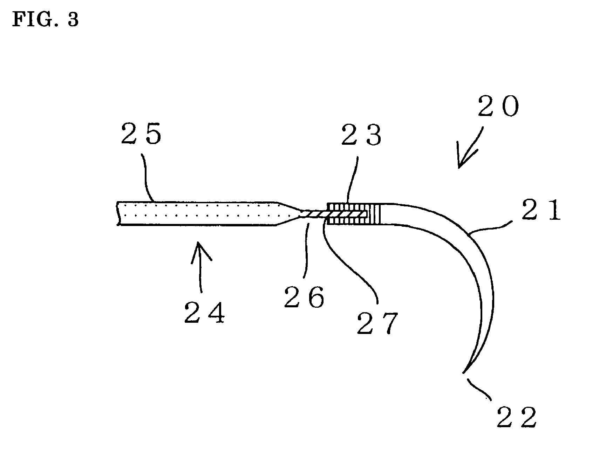

A surgical needle generally has an eye provided on the cross-section of the dull edge for attaching a suture (see Figs. 2 and 3). An end part of a suture is inserted in this hole and is bonded by caulking or the like. Therefore, the diameter of the suture must essentially be smaller than the diameter of the dull edge of the surgical needle.

Hitherto, a method of producing a monofilament suture having an end part with a small diameter has been proposed. In the method, a part of a monofilament suture formed using a synthetic resin such as polypropylene is heated at a high temperature, the heated part is expanded to decrease the diameter of the expanded part, and the expanded part is then cut. See, for example, Japanese Unexamined Patent Application Publication No. 3-210251. According to this method, since a part of the suture having a small diameter can be bonded with an eye of a surgical needle, the diameter of the dull edge of the needle and the diameter of the suture can be practically adjusted so as to be the same.

The above expanding method can be applied to a monofilament suture composed of a thermoplastic resin such as polypropylene. However, in the case where this method is applied to a monofilament suture composed of a porous expanded PTFE, only the porosity of the expanded part is increased and the decreasing ratio of the cross-section is small. Increasing the drawing ratio tends to break the expanded part. In addition, when the end part having an increased porosity due to the expansion (i.e., drawing) is inserted in the hole formed by perforating the cross-section of the dull edge of a surgical needle, the suture is too soft to be bonded firmly by caulking.

For example, Japanese Unexamined Patent Application Publication No. 3-289962 proposes a method of reducing the diameter of a monofilament suture composed of a porous expanded PTFE. In the method, an unsintered rod-shaped article is formed by a paste extrusion of PTFE. The resultant formed article is expanded at a temperature lower than or equal to the melting point, sintered, and subsequently, subjected to a die drawing. According to this method of die drawing, a monofilament suture with an improved tensile strength in which only the outer periphery is nonporous can be produced.

However, in order to produce sutures with various dimensions of reduced diameter by the above method, a large number of dies is required to obtain the desired dimensions. In addition, the dimension of reduced diameter depends on the porosity of the original porous expanded PTFE. Also, with this method, it is difficult to obtain a desired suture because it is difficult to handle an expanded PTFE monofilament having a small diameter of 200 µm or less for stitching blood vessels.

Furthermore, in the above method, the porous structure is maintained inside the monofilament suture. Therefore, it is difficult to significantly reduce the diameter and it is difficult to reduce the diameter of only an end part of monofilament suture. Furthermore, even if the diameter of the end part of the monofilament suture is reduced, a strong bonding by caulking with the hole of a surgical needle cannot be expected because the end part is soft.

According to Japanese Unexamined Patent Application Publication No. 11-116713, the diameter of at least a part of a rod-shaped porous expanded PTFE such as a monofilament suture is reduced by providing a twist deformation. According to this method, since the volume is reduced while fibrils in the porous structure are intertwisted, the entire part of the rod-shaped article to which the twist deformation is provided is contracted in the radius direction. Thus, a reduction in porosity and a reduction in diameter are achieved at the same time.

In the above method, after the twisting process, the rod-shaped article is heated at a temperature higher than or equal to the melting point of PTFE in a state where the length is not changed (i.e., in a stretched state) to fix the twist deformation. When the porosity of the part that has been subjected to the twist deformation is sufficiently decreased, an expanded PTFE formed article having a semitransparent high density part with a reduced diameter can be produced by heating fixation. In this method, the diameter can be further decreased not only by the twist deformation but also by expanding the part that has been subjected to the twist deformation.

According to the method disclosed in Japanese Unexamined Patent Application Publication No. 11-116713, an expanded PTFE formed article having a substantially nonporous part with a reduced diameter can be obtained by subjecting at least a part of a rod-shaped porous expanded PTFE to a twist deformation and performing a heating fixation, or alternatively, expanding the porous expanded PTFE at a high temperature in addition to the twist deformation.

This method allows the diameter of an end part of a monofilament suture composed of a porous expanded PTFE to be reduced. Therefore, it is possible to insert the end part having a reduced diameter into an eye of the dull edge of a surgical needle and to bond, by caulking, the monofilament suture with a surgical needle having the dull edge with a diameter smaller than or equal to the diameter of the suture.

However, PTFE itself is a resin material having a low tensile elastic modulus and being readily subjected to a plastic deformation. Therefore, in such a monofilament suture composed of a porous expanded PTFE, the substantially nonporous part having a reduced diameter also has a low elastic modulus and is readily subjected to a plastic deformation. Accordingly, it is difficult to achieve a satisfactory bonding strength even when the monofilament suture is inserted in the eye of a surgical needle and bonded by caulking. Also, it is desired that the tensile strength of the part with a reduced diameter be further improved.

### Disclosure of Invention

An object of the present invention is to provide an expanded PTFE formed article in which a part having a diameter reduced by a twist deformation and having high tensile strength and elastic modulus is provided to at least a part (i.e., the whole or a part) of a porous expanded PTFE rod-shaped article. Also, an object of the present invention is to provide a method of producing an expanded PTFE formed article including a part having a diameter reduced by a twist deformation and having high tensile strength and elastic modulus.

Another object of the present invention is to provide a composite article comprising a metal wire and an expanded PTFE formed article including a part having a diameter reduced by twist deformation and having high tensile strength and elastic modulus, wherein the diameter reduced end part of the expanded PTFE formed article is inserted in a hole formed by perforating the cross-section of an end part of the metal wire. In the composite article, an end part of the formed article composed of the expanded PTFE, the end part having a reduced diameter, is inserted in a hole formed by perforating the cross-section of an end part of the metal wire.

In particular, an object of the present invention is to provide a composite article comprising a surgical needle and a porous expanded PTFE monofilament suture, wherein a diameter reduced end part of the monofilament suture is inserted in a hole formed by perforating the cross-section of the dull edge of the surgical needle and the bonding strength between the needle and the suture is satisfactorily increased by caulking.

In order to achieve the above objects, the inventors of the present invention have conducted intensive studies and found the following: in a method for reducing the diameter of at least a part of a porous expanded PTFE rod-shaped article by providing a twist deformation thereto, an expanded PTFE formed article in which a substantially nonporous part having a reduced diameter has high tensile strength and in which the bonding strength with a needle is significantly increased can be produced by performing the twist deformation after applying or impregnating a synthetic resin having a tensile elastic modulus higher than the tensile elastic modulus (a value measured at normal temperature) of PTFE onto a part to which the twist deformation is to be provided. The present invention has been completed on the basis of the above finding.

The present invention provides an expanded polytetrafluoroethylene formed article wherein a synthetic resin having a tensile elastic modulus higher than the tensile elastic modulus of polytetrafluoroethylene is applied on or impregnated into at least a part of a porous expanded polytetrafluoroethylene rod-shaped article having a porous structure composed of fibrils and nodes connected by fibrils, and wherein the diameter of the part to which the synthetic resin has been applied or impregnated is reduced by a twist deformation.

The present invention also provides a method of producing an expanded polytetrafluoroethylene formed article, comprising the steps of (1) applying or impregnating a synthetic resin having a tensile elastic modulus higher than the tensile elastic modulus of polytetrafluoroethylene to at least a part of a porous expanded polytetrafluoroethylene rod-shaped article having a porous structure consisting of fibrils and nodes connected by fibrils, and (2) providing a twist deformation so as to reduce the diameter of the part to which the synthetic resin has been applied or impregnated.

Furthermore, the present invention provides a composite article comprising (a) an expanded polytetrafluoroethylene formed article in which a synthetic resin having a tensile elastic modulus higher than the tensile elastic modulus of polytetrafluoroethylene is applied on or impregnated into an end part of a porous expanded polytetrafluoroethylene rod-shaped article having a porous structure consisting of fibrils and nodes connected by fibrils and in which the diameter of the end part is reduced by a twist deformation and (b) a metal wire, wherein the diameter reduced end part of the expanded polytetrafluoroethylene formed article is inserted in a hole perforated on the cross-section of an end part of the metal wire.

The present invention provides an expanded PTFE article which is formed such that a part having a diameter reduced by a twist deformation and having high tensile strength and elastic modulus is provided to at least a part of a porous expanded PTFE rod-shaped article. Also, the present invention provides a method of producing an expanded PTFE formed article including a part having a diameter reduced by twist deformation and having high tensile strength and elastic modulus.

The present invention also provides a composite article comprising a metal wire and an expanded PTFE formed article that includes a part having a diameter reduced by a twist deformation and having high tensile strength and elastic modulus, wherein the diameter reduced end part of the expanded PTFE formed article is inserted in a hole perforated on the cross-section of an end part of the metal wire. In the composite article, the end part having the reduced diameter of the formed article composed of expanded PTFE is inserted in a hole perforated on the cross-section of an end part of the metal wire. In particular, the present invention provides a composite article comprising a monofilament suture and a surgical needle, wherein an end part having a reduced diameter of the porous expanded PTFE monofilament suture is inserted in a hole perforated on the cross-section of the dull edge of the surgical needle, and wherein the bonding strength thereof with the needle is satisfactorily increased by caulking.

### Brief Description of the Drawings

Figure 1 is a schematic cross-sectional view showing an example of a formed article composed of expanded PTFE of the present invention.
Figure 2 is a schematic view showing a state in which a part having a reduced diameter of a monofilament suture of the present invention is inserted in and bonded with the eye of a surgical needle.
Figure 3 is a schematic view (including a cross-sectional view) showing a state in which a diameter reduced part of a monofilament suture of the present invention is inserted in and bonded with the eye of a surgical needle.

### Best Mode for Carrying Out the Invention

The term "porous expanded polytetrafluoroethylene rod-shaped article having a porous structure consisting of fibrils and nodes connected by the fibrils" or the like as used in the present invention means an article which falls within a scope from a narrow bar-shaped formed article (diameter: about 1 to about 10 mm) to a monofilament (diameter: about 10 to about 1,000 µm). The cross-sectional shape of the rod-shaped article is generally a round shape, but may be an elliptical shape, a polygon, or the like as required.

In a case where the rod-shaped article has an excessively large diameter, the formed article is easily broken when a twist deformation is provided. A rod-shaped article to be used for an application of a medical suture is a monofilament. The diameter of the monofilament is selected from a range that can be applied to a suture and is generally about 400 µm or less, preferably about 80 to about 300 µm, and more preferably about 100 to about 250 µm. The length of the rod-shaped article can be appropriately determined according to the application.

A rod-shaped article can be generally produced as follows (for example, Japanese Examined Patent Application Publication No. 42-13560): A liquid lubricant is mixed with an unsintered PTFE powder; the mixture is extruded by ram extruding so as to have a relatively thick rod shape; subsequently, the resultant rod is expanded in the axial direction in an arbitrary drawing ratio providing strain and strain rate; and the rod is then heated above 327°C, which is a sintering temperature of PTFE, or higher while the rod is fixed so as not to cause a contraction, so that the expanded structure is fixed by sintering.

By this expanding method, a rod-shaped article can be produced as a porous expanded PTFE article having a micro fibrous structure (porous structure) in which a large number of fibrils (micro fibers) are connected with each other by a large number of nodes (micro nodes). The average pore size, the porosity, the average length of the fibril, and the like of the porous expanded PTFE can be controlled in a desired range by adjusting the drawing ratio providing strain and strain rate. When the rod-shaped article is used for an application of a medical suture, the average length of the fibril is 5 to 90 µm, preferably 60 to 90 µm, and the porosity is generally 40% or more, preferably 50% to 80%.

In the present invention, a synthetic resin having a tensile elastic modulus higher than the tensile elastic modulus of PTFE is applied on or impregnated into at least a part of a rod-shaped article composed of a porous expanded PTFE. Subsequently, the diameter of the part to which the synthetic resin is applied or impregnated is reduced by a twist deformation. In the reduction of the diameter, an expansion by drawing is preferably performed in addition to the twist deformation. When the overall diameter of the rod-shaped article is to be reduced, the synthetic resin is applied on or impregnated into the entire part of the rod-shaped article. As in a suture, when a part having a small diameter for connecting to an eye of a surgical needle is required, the synthetic resin is applied on or impregnated into a part of the rod-shaped article.

In general, the tensile elastic modulus of PTFE at normal temperature (23°C) is relatively small, about 390 to about 550 MPa, and a plastic deformation can easily be made. Apart where the diameter has been reduced by a twist deformation is also easily subject to plastic deformation and has low tensile strength. Accordingly, in the present invention, a synthetic resin having a tensile elastic modulus higher than the tensile elastic modulus of PTFE is applied on or impregnated into at least a part of a rod-shaped article.

Such synthetic resins are, for example, acrylic resins, methacrylic resins, epoxy resins, polyolefins (such as polyethylene and polypropylene), polyimides, polyamideimides, polyacetal, polycarbonate, polyphenylene ether, polyamides, polyphenylene sulfide, polyether sulfone, polyvinyl chloride, polystyrene, styrene-acrylonitrile copolymer (SAN resin), acrylonitrile-butadiene-styrene resin (ABS resin), etc

Among these synthetic resins, synthetic resins having a sufficiently high tensile elastic modulus of preferably at least 1,000 MPa, and more preferably at least 1,500 MPa are desirable. For example, polymethyl methacrylate (PMMA) (tensile elastic modulus = about 2,240 to 3,240 MPa), epoxy resins (tensile elastic modulus = about 2,410 MPa), and polyimides (tensile elastic modulus = about 2,070 MPa) are preferable. However, the values of tensile elastic modulus described herein are just typical values. The synthetic resins are not particularly limited to resins having a tensile elastic modulus in this numerical range.

In order to apply or impregnate these synthetic resins, a solution prepared by dissolving a synthetic resin (or its precursor) in a solvent is generally used. In order that such a solution is applied on the surface of a porous expanded PTFE rod-shaped article or impregnated into a porous structure thereof, the solvent preferably has an affinity with the porous expanded PTFE. When a synthetic resin soluble in a solvent having an affinity with the porous expanded PTFE is used, the application or the impregnation can be readily performed. Consequently, the synthetic resin satisfactorily permeates on the surface or in the porous structure of the rod-shaped article. Examples of the solvent include halogenated hydrocarbon solvents such as dichloromethane, ketone solvents such as acetone, and aromatic hydrocarbon solvents such as xylenes.

For example, since methacrylic resins such as polymethyl methacrylate (PMMA) are soluble in solvents such as dichloromethane and acetone, which have an affinity with the porous expanded PTFE, the use of a solution containing these can control the permeability. In addition, for example, since precursors of polyimide (polyamic acids) are soluble in solvents such as xylenes, which have an affinity with the porous expanded PTFE, the use of a solution containing these can control the permeability.

The optimum concentration of synthetic resin (or its precursor) in the solution can be appropriately experimentally determined so that the strength of the part having a reduced diameter is increased, while the permeability on the surface or in the porous structure of a rod-shaped article composed of a porous expanded PTFE is increased. The concentration of synthetic resin in the solution is preferably 0.1 to 15 percent by weight, more preferably 0.5 to 10 percent by weight, and particularly preferably 1 to 5 percent by weight. If the concentration is excessively low, the workability is impaired, and it is difficult to apply or impregnate a sufficient amount of synthetic resin. On the other hand, if the concentration is excessively high, the permeability is decreased depending on the kind of synthetic resins, which tends to result in difficulty of impregnating a sufficient amount of synthetic resin into a porous structure. The amount of impregnation can be controlled by adjusting the concentration of the solution.

The solution of synthetic resin is applied on or impregnated into a desired part of a rod-shaped article. However, it is preferable to impregnate the solution into the entire diameter reduced part of porous structure in order to change the diameter reduced part to be nonporous so as to increase the tensile strength and in order to increase the bonding strength between the diameter reduced part of a suture and the eye of a surgical needle. In contrast, when maintaining the porous structure is preferred, the solution may be applied on the surface of a rod-shaped article so that the solution is impregnated into only a surface layer part of the porous structure.

After the solution of synthetic resin is applied on or impregnated into the desired part of the rod-shaped article, the formed article is dried to remove the solvent. The drying may be either a natural drying in air or a heated drying in a heating furnace. When the rod-shaped article contracts in the longitudinal direction during drying, the formed article is preferably dried in a fixed state (in a stretched state) so as not to contract. The synthetic resin is precipitated on the surface or in the porous structure of the rod-shaped article by drying.

When a heat treatment is performed at a temperature higher than or equal to the melting point of the synthetic resin after or during the drying, the synthetic resin integrates with the porous expanded PTFE article by being solidified or hardened in the porous structure. This heating treatment is effective when the synthetic resin is a thermoplastic resin such as PMMA. The temperature of the heat treatment is generally a temperature which is equal to or higher than the melting point of the synthetic resin and which is lower than the thermal decomposition temperature of the synthetic resin. The duration of the heat treatment is generally about 1 minute to about 10 hours, preferably about 10 minutes to about 5 hours, and in most cases about 0.5 to about 3 hours. The heat treatment is generally performed before the process of reducing the diameter by twisting deformation or expansion. However, the heat treatment may be performed after the process of reducing the diameter, or alternatively according to need, before and after the process of reducing the diameter. The heating treatment can improve the tensile strength of the part having a reduced diameter.

On the other hand, when a precursor such as a polyamide acid or a thermosetting resin such as an epoxy resin is used as a material of the synthetic resin, the heat treatment is performed after the process of reducing the diameter by the twisting deformation or expansion. For example, the polyamic acid is subjected to a dehydration ring closing (imidization) by the heat treatment to form a polyimide. With respect to the conditions of this heat treatment, a suitable heating temperature and duration of heat treatment are selected so that imidization or thermal curing reaction may occur under the conditions.

In the present invention, after a synthetic resin having a high tensile elastic modulus is applied on or impregnated into at least a part of a porous expanded PTFE rod-shaped article, a twist deformation is provided to the part so as to reduce the diameter. For example, a method for providing a twist deformation is such that a twist of at least 1 rotation/cm is provided to at least a part of the rod-shaped article and thereafter the status of twist deformation is fixed by heating at a temperature higher than or equal to the melting point of PTFE. More specifically, examples of the method of providing a twist deformation include (i) a method in which both ends of a rod-shaped article are held by grips and one of the grips is rotated while the other grip is fixed, and (ii) a method in which a drum is rotated in a direction such that a rod-shaped article is twisted while the rod-shaped article is wound on the drum. These methods are similar to methods for twisting a fiber, and the same methods can be employed. When a twist deformation is provided to a part of a rod-shaped article, the same method as the above is applied to the part. A twist deformation is generally provided by rotating a desired part of a rod-shaped article in one direction to twist the part.

The degree of twist deformation can be controlled by adjusting the number of rotations. In the method of the present invention, a twist of at least 1 rotation/cm is provided to at least a part of a rod-shaped article. The number of rotations can be appropriately selected according to the diameter of the rod-shaped article or the desired degree of reduction in diameter. When a medical suture is produced using an expanded PTFE monofilament as a rod-shaped article, the number of rotations is generally about 1 to about 250 rotation/cm, preferably about 10 to about 200 rotation/cm, and more preferably about 15 to about 150 rotation/cm. The number of rotations within the above range allows the diameter to be reduced to a desired dimension.

When a twist deformation is applied to a rod-shaped article, the entire part thereof contracts in a radius direction since the volume is reduced as a result of intertwisting of fibrils in the micro fibrous structure constituting a porous body. Thus, a reduction in porosity and a reduction in diameter are caused at the same time. After the process of applying a twisting deformation, the rod-shaped article is subjected to heat treatment in a fixed state where the length of the formed article is not changed (in a stretched state), whereby the status of the twist deformation is fixed. The temperature of this heat treatment (the temperature of heating fixation) is a temperature which is higher than or equal to the melting point of a synthetic resin or higher than or equal to the hardening temperature of a synthetic resin and which is lower than the thermal decomposition temperature of the synthetic resin. When the thermal decomposition temperature of the synthetic resin is excessively high, the temperature of the heat treatment is set to a temperature lower than the thermal decomposition temperature of PTFE. The heat treatment for heating fixation may be performed at a temperature higher than or equal to the melting point of PTFE. The temperature of heating fixation is preferably about 150°C to about 380°C. The duration of heating fixation is generally about 10 seconds to about 30 minutes, preferably about 1 to about 10 minutes. When the porosity of a part that has been subjected to the twist deformation is sufficiently decreased, an expanded PTFE formed article having a diameter reduced part which is made to be semitransparent and of high density by heating fixation can be obtained, depending on the degree or the number of rotations of twist deformation, or depending on the degree of expansion, which will be described below. The entire part of the rod-shaped article can be processed to have a reduced diameter with high density in such a manner. In addition, when a desired reduction in diameter can be achieved, a porous state can be maintained without excessively decreasing the porosity.

The expanded PTFE formed article produced as described above generally has spiral wrinkles formed on the outer periphery of a part having a reduced diameter (see Figs. 1 and 2 in Japanese Unexamined Patent Application Publication No. 11-116713). That is, spiral wrinkles (fine muscles or folds) are formed in the twisted direction on the expanded PTFE article thus produced. In the case where a medical suture is produced with a monofilament, the crossing angle formed by the wrinkles (muscles or folds) and the major axis of the expanded PTFE formed article is generally 10 degrees or more, preferably 11 degrees or more. By increasing this crossing angle, the porosity of a part having a reduced diameter can be sufficiently decreased and the tensile strength can be increased. The upper limit of crossing angle is generally about 60 degrees, preferably about 50 degrees, and more preferably about 40 degrees.

When twist deformation is performed after a synthetic resin having a high tensile elastic modulus has been applied on or impregnated into a desired part of a rod-shaped article, the volume is reduced with the intertwisting of fibrous structures constituting a porous structure, and accordingly the entire structure contracts in the radius direction. Thus, a reduction in porosity and a reduction in diameter are caused at the same time. Subsequently, by performing heat treatment so as to solidify or harden the synthetic resin applied or impregnated, an expanded PTFE article having a partially hardened part with a reduced diameter whose shape is fixed can be produced.

In the present invention, a twist deformation is applied to at least a part of a rod-shaped article, and in addition, the part can be expanded. Since the rod-shaped article has already been expanded in the production process, the expansion in the reduction in diameter is also referred to as a secondary expansion. Two methods are employed for providing a twist deformation and an expansion: In a first method, a twist deformation is provided to at least a part of a rod-shaped article, and a secondary expansion is then performed at a temperature of 60°C or higher, and a heating fixation is then performed. In a second method, a secondary expansion is performed while a twist deformation is provided to at least a part of a rod-shaped article at a temperature of 60°C or higher, and a heating fixation is then performed.

In the method of the present invention, since a synthetic resin is applied on or impregnated into a desired part of a rod-shaped article, the secondary expansion can be performed at a relatively low temperature. Although the expanding temperature (i.e., drawing temperature) is different depending on the type of the synthetic resin used, the temperature is determined from a range of, generally 60°C to 380°C, preferably 80°C to 300°C. At an excessively high expanding temperature, the expanded part is easily broken. In order to perform the expansion, for example, in the above method for providing a twist deformation, the grips should be moved so as to be distanced from each other, or when a drum is used, the winding speed should be higher than the supply speed.

The drawing ratio can be appropriately determined according to a desired degree of the reduction in diameter. The drawing ratio is generally 10% to 1,000%, preferably 20% to 500%, and more preferably 30% to 300%. By performing the expansion by drawing in addition to the twist deformation, the degree of the reduction in diameter can be increased. When the drawing ratio is 50% or more, in most cases, the outer diameter of an expanded PTFE formed article that has been subjected to the twist deformation and the expansion by drawing can be reduced to about 1/2 or less of the outer diameter of an original rod-shaped article. The expanded state can be fixed by performing a heat treatment after the expansion process at a temperature higher than or equal to the melting point of the synthetic resin or at a temperature higher than or equal to the hardening temperature of the synthetic resin. The temperature of this heating fixation is lower than the thermal decomposition temperatures of the synthetic resin and PTFE.

Also in the case where both a twist deformation and an expansion are provided, it is possible to see, by a microscopic observation, spiral wrinkles formed on the outer periphery of a part having a reduced diameter of the resultant expanded PTFE formed article. The above-described crossing angle in this case should be generally 1 degree or more and preferably 2 degrees or more, from the viewpoint of sufficiently decreasing the porosity of a part having a reduced diameter and increasing the tensile strength. In this case, the upper limit of crossing angle is about 10 degrees. The expanded PTFE formed article of the present invention may have a reduced diameter in either the entire part or a partial part thereof according to need. Likewise, the expanded PTFE formed article of the present invention may be provided with a partial twist deformation or expansion in addition to a twist deformation totally applied, or instead, may be provided with a partial deformation and a partial expansion. Alternatively, a partial deformation and a partial expansion may further be provided to the formed article.

The diameter of a part having a reduced diameter of a rod-shaped article is preferably about 20% to about 80%, more preferably about 30% to about 70%, and particularly preferably about 35% to about 60% of the diameter of the original rod-shaped article.

In the case where the diameter of a part of a rod-shaped article is to be reduced, such part may be an end or an arbitrarily selected part of the rod-shaped article. The part to be expanded by drawing may also be the entire part or the central portion of the part to which a twist deformation has been provided. By cutting a monofilament at the part having a reduced diameter, a monofilament having an end with a small diameter can be obtained. When this monofilament is used as a suture, the end having the small diameter of the monofilament can be bonded with an eye (or a groove) on the dull edge of a needle

Figure 1 is a schematic cross-sectional view of an expanded PTFE formed article 1, as obtained by cutting the part where the diameter is partially reduced. The expanded PTFE formed article 1 includes a part 2 in which a porous structure is maintained without being subjected to a twist deformation and an expanding treatment, and a part 3 having a reduced diameter which exists at an end of the formed article 1.

In the case where a monofilament having the structure shown in Fig. 1 is used as a suture, a composite article comprising a monofilament suture and a surgical needle can be obtained, as shown in Figs. 2 and 3, by inserting a diameter reduced part 26, which is formed at an end of a main part 25 of a monofilament 24, into a hole formed by perforating the cross-section of a dull edge 23 of a surgical needle 20. When the diameter of the eye of the surgical needle is reduced by caulking after the diameter reduced end part of the monofilament suture has been inserted in the eye, the surgical needle can be closely integrated with the diameter reduced part and thereby their bonding strength can be increased. In the surgical needle, the dull edge 23 having a large diameter is disposed at one end of a main part 21, and a sharp edge (needle point) 22 is disposed at the opposite end. The eye provided on the dull edge of the surgical needle may partially include a groove cut along the side face of the dull edge.

In the composite article having the above-described structure made by combining a monofilament suture and a surgical needle, the diameter of the suture is controlled so as to be approximately the same as or slightly larger than the diameter of the dull edge of the surgical needle so that holes in the living body tissues formed by the dull edge of the surgical needle can be filled with the suture. Accordingly, problems such as the leakage of blood can be eliminated.

The composite article made of a monofilament suture and a surgical needle can be used in a surgical operation or the like as it is. In addition, the technique for making such a composite article can be applied to not only the field of sutures but also generally to forming a composite article by combining a metal wire and a expanded PTFE formed article including a part having a reduced diameter.

### Examples

The present invention will now be described more specifically together with examples and comparative examples. Methods of evaluating the physical properties are as follows:

### (1) Tensile test:

A tensile test of a part that was subjected to a process of reducing the diameter was performed under the following conditions to determine the maximum tensile load and the tensile strength.
Tester: Autograph AG500E from Shimadzu Corporation
Initial length of sample: 50 mm (the distance between grips)
Number of samples: 3 (n = 3)
Tensile speed: 50 mm/min (initial strain rate 100%/min)
Tensile strength (average): maximum load/cross-sectional area (kgf/mm²)

### (2) Effect of making nonporous

The results of visual observation of the leading end portion of diameter reduced parts of expanded PTFE monofilaments were determined as follows: when the appearance of the end portion was changed to be semitransparent, the effect of making nonporous existed; when the appearance of the end was still opaque, the effect of making nonporous did not exist.
A: The appearance was changed to be semitransparent.
B: The appearance was still opaque.

### (3) Test of pull-out strength at the part bonded with a needle

A stainless steel needle having an outer diameter of 140 µm and having a hole of 80 µm in inner diameter perforated on the dull edge thereof was prepared. The end portion of a diameter reduced part of an expanded PTFE monofilament, which end portion was cut with an edged knife, (i.e., the end portion having the reduced diameter) was inserted in the perforated hole part of the needle. A pressure was applied from the outer periphery of the dull edge of the needle so that the inner diameter was contracted by caulking. Thus, the leading end of the expanded PTFE monofilament was bonded with the dull edge of the needle, and consequently a suture with a needle was prepared. The needle part was fixed to a needle holder, and a weight of 50 g was vertically suspended to apply a pull-out load. The number of samples (n) is shown in a Table. The bonding strength is changed according to the strength of the part bonded by caulking, that is, the thickness of the needle (the outer diameter of the dull edge of needle/the inner diameter of the dull edge of needle) or the like. In this test, since a needle corresponding to the U.S.P. standard 8-0 was used, the weight of 50 g was used.

### (EXAMPLE 1)

A paste was prepared by blending a PTFE fine powder (trade name, CD123) from Asahi Glass Co., Ltd. with 14 parts by weight of tetradecane, serving as a lubricant, from Wako Pure Chemical Industries, Ltd. This paste was extruded to prepare a rod-shaped extruded article under the following extrusion conditions: the diameter of a cylinder was 14 mm; the diameter of a die was 0.3 mm; the extrusion temperature was 110°C; and the ram speed was 0.5 mm/min. The resultant rod-shaped extruded article was dried for 48 hours in a hot air circulation oven heated at 80°C so that the tetradecane was removed. The extruded article was supplied into a radiant oven having a length of 450 mm wherein the atmospheric temperature was controlled to about 400°C, and the extruded article was sintered simultaneously while the extruded article was continuously expanded with a drawing ratio of 500%. As a result, an expanded PTFE monofilament having an outer diameter of 169 µm and a weight of 0.00656 g/30 cm was obtained. The resultant expanded PTFE monofilament was a porous PTFE monofilament having an apparent specific gravity of 0.975 g/m, which was calculated from its volume and weight, and a porosity of 57%, which was calculated from the true specific gravity of PTFE (2.25 g/ml).

A polymethyl methacrylate (PMMA; trade name, SUMIPEX LG6A) from Sumitomo Chemical Co., Ltd. (1 g) was dissolved in dichloromethane (99 g) to prepare a 1% PMMA solution. An end part (10 cm) of the expanded PTFE monofilament (100 cm in length) was immersed in the PMMA solution so that the PMMA solution was impregnated into the porous structure. Subsequently, the excessive solution was wiped out and the monofilament was naturally dried.

The part (10 cm) of the expanded PTFE monofilament impregnated with PMMA was twisted at a rate of 300 times/min for 15 seconds. Subsequently, the central part of the above part (10 cm) was expanded while the central part was heated with a flat heater plate (13 mm in width) heated at 110°C. Thus, only the central part was processed to be semitransparent while the outer diameter of the part was controlled to about 70 µm. By the above method, an expanded PTFE monofilament including a part having a reduced diameter of 72 µm and a main part having an outer diameter of 169 µm was produced.

### (EXAMPLE 2)

The PMMA (SUMIPEX LG6A) from Sumitomo Chemical Co., Ltd. (2 g) was dissolved in dichloromethane (98 g) to prepare a 2% PMMA solution. An end part (10 cm) of the expanded PTFE monofilament (100 cm in length) was immersed in the PMMA solution so that the PMMA solution permeated into the monofilament. Subsequently, the excessive solution was wiped out and the monofilament was naturally dried. The same process was performed as in Example 1 except for the above, and consequently an expanded PTFE monofilament including a part having a reduced diameter of 72 µm was produced.

### (EXAMPLE 3)

The PMMA (SUMIPEX LG6A) from Sumitomo Chemical Co., Ltd. (3 g) was dissolved in dichloromethane (97 g) to prepare a 3% PMMA solution. An end part (10 cm) of the expanded PTFE monofilament (100 cm in length) was immersed in the PMMA solution so that the PMMA solution was impregnated into the monofilament. Subsequently, the excessive solution was wiped out and the monofilament was naturally dried. The same process was performed as in Example 1 except for the above to produce an expanded PTFE monofilament including a part having a reduced diameter of 72 µm.

### (EXAMPLE 4)

The PMMA (SUMIPEX LG6A) from Sumitomo Chemical Co., Ltd. (4 g) was dissolved in dichloromethane (96 g) to prepare a 4% PMMA solution. An end part (10 cm) of the expanded PTFE monofilament (100 cm in length) was immersed in the PMMA solution so that the PMMA solution was impregnated into the monofilament. Subsequently, the excessive solution was wiped out and the monofilament was naturally dried. The same process was performed as in Example 1 except for the above to produce an expanded PTFE monofilament including a part having a reduced diameter of 70 µm.

### (EXAMPLE 5)

The PMMA (SUMIPEX LG6A) from Sumitomo Chemical Co., Ltd. (5 g) was dissolved in dichloromethane (95 g) to prepare a 5% PMMA solution. An end part (10 cm) of the expanded PTFE monofilament (100 cm in length) was immersed in the PMMA solution so that the PMMA solution was impregnated into the monofilament. Subsequently, the excessive solution was wiped out and the monofilament was naturally dried. The same process was performed as in Example 1 except for the above to produce an expanded PTFE monofilament including a part having a reduced diameter of 71 µm.

### (EXAMPLE 6)

The PMMA (SUMIPEX LG6A) from Sumitomo Chemical Co., Ltd. (1 g) was dissolved in dichloromethane (99 g) to prepare a 1% PMMA solution. An end part (10 cm) of the same expanded PTFE monofilament (100 cm in length) as that in Example 1 was immersed in the PMMA solution so that the PMMA solution was impregnated into the monofilament. Subsequently, the excessive solution was wiped out and the monofilament was naturally dried. Subsequently, the expanded PTFE monofilament was heated at 190°C for 2 hours in a hot air circulation oven while the monofilament was fixed so as not to be contracted in the longitudinal direction.

The part (10 cm) of the expanded PTFE monofilament that was impregnated with PMMA and subjected to the heat treatment was twisted at a rate of 300 times/min for 15 seconds. Subsequently, the central part of the above-mentioned part was expanded while the central part was heated with a flat heater plate (13 mm in width) at 110°C. Accordingly, while only the central part was changed to be semitransparent, the outer diameter of the part was controlled to about 70 µm. By the above method, an expanded PTFE monofilament including a part having a reduced diameter of 75 µm and a main part having an outer diameter of 169 µm was produced.

### (EXAMPLE 7)

The PMMA (SUMIPEX LG6A) from Sumitomo Chemical Co., Ltd. (2 g) was dissolved in dichloromethane (98 g) to prepare a 2% PMMA solution. An end part (10 cm) of the same expanded PTFE monofilament (100 cm in length) as that in Example 1 was immersed in the PMMA solution so that the PMMA solution was impregnated into the monofilament. Subsequently, the excessive solution was wiped out and the monofilament was naturally dried. The same process was performed as in Example 6 except for the above to produce an expanded PTFE monofilament including a part having a reduced diameter of 74 µm and a main part having an outer diameter of 169 µm.

### (EXAMPLE 8)

The PMMA (SUMIPEX LG6A) from Sumitomo Chemical Co., Ltd. (3 g) was dissolved in dichloromethane (97 g) to prepare a 3% PMMA solution. An end (10 cm) of the same expanded PTFE monofilament (100 cm in length) as that in Example 1 was immersed in the PMMA solution so that the PMMA solution was impregnated into the monofilament. Subsequently, the excessive solution was wiped out and the monofilament was naturally dried. The same process was performed as in Example 6 except for the above to produce an expanded PTFE monofilament including a part having a reduced diameter of 71 µm and a main part having an outer diameter of 169 µm.

### (EXAMPLE 9)

The PMMA (SUMIPEX LG6A) from Sumitomo Chemical Co., Ltd. (4 g) was dissolved in dichloromethane (96 g) to prepare a 4% PMMA solution. An end part (10 cm) of the same expanded PTFE monofilament (100 cm in length) as that in Example 1 was immersed in the PMMA solution so that the PMMA solution was impregnated into the monofilament. Subsequently, the excessive solution was wiped out and the monofilament was naturally dried. The same process was performed as in Example 6 except for the above to produce an expanded PTFE monofilament including a part having a reduced diameter of 68 µm and a main part having an outer diameter of 169 µm.

### (COMPARATIVE EXAMPLE 1)

The central part of an end part (10 cm) of the expanded PTFE monofilament prepared in Example 1, which had an outer diameter of 169 µm, was expanded while the central part was heated with a flat heater plate (13 mm in width) at 350°C. However, the filament was broken before the outer diameter reached about 70 µm. The appearance of the heated part of the expanded PTFE monofilament remained opaque.

### (COMPARATIVE EXAMPLE 2)

An end part (10 cm) of the expanded PTFE monofilament prepared in Example 1, which had an outer diameter of 169 µm, was twisted at a rate of 300 times/min for 15 seconds. Subsequently, the central part of the above part (10 cm) was expanded while the central part was heated with a flat heater plate (13 mm in width) at 110°C. Thus, the outer diameter of the part was controlled to about 70 µm while only the central part was changed to be semitransparent. By this method, an expanded PTFE monofilament including a leading end having a reduced diameter of 72 µm and a main part having an outer diameter of 169 µm was produced.

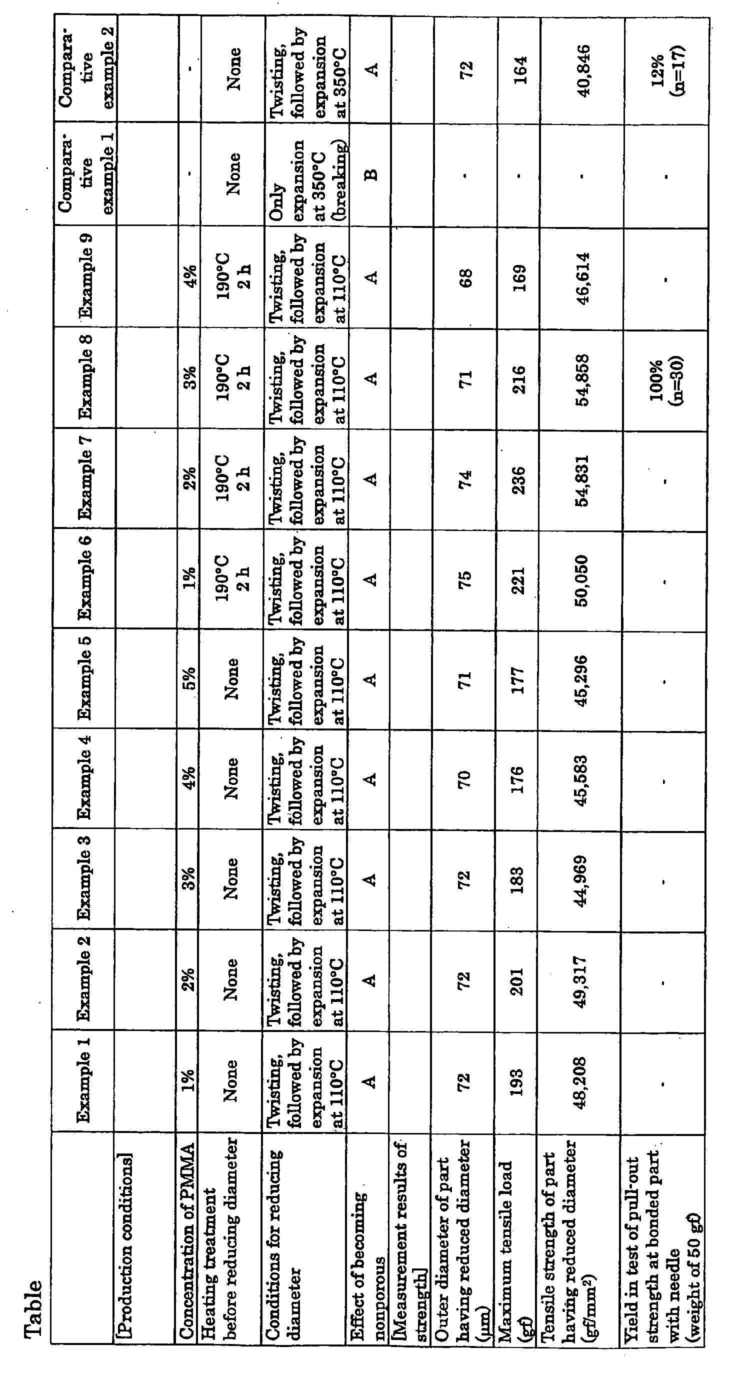

As is apparent from the results shown in the Table, the expanded PTFE monofilaments each having a leading end that was impregnated with PMMA and was then subjected to a process of reducing the diameter by a twist deformation and an expansion (Examples 1 to 9) had a higher tensile strength and a superior pull-out strength at the part bonded with a surgical needle, and thus exhibited superior characteristics as a suture, as compared with the expanded PTFE monofilament that was subjected to a process of reducing the diameter without the impregnation of PMMA (Comparative Example 2).

In addition, the tensile strength of the expanded PTFE monofilaments each having a leading end that was impregnated with PMMA and was then subjected to a heat treatment prior to the process of reducing the diameter (Examples 6 to 9) tended to be higher than that of the expanded PTFE monofilaments that were not subjected to the corresponding heat treatment (Examples 1 to 4). The concentration of PMMA is preferably 5% or less, more preferably 1% to 4%, and particularly preferably 2% to 3%. The increase in the concentration of PMMA increases the viscosity of the solution. It is assumed that the increase in the viscosity of the solution is related to the decrease in the permeability into the porous structure of an expanded PTFE monofilament. On the other hand, in the case where an end of expanded PTFE monofilament was simply expanded without the impregnation of PMMA (Comparative Example 1), the monofilament was broken and the diameter could not be reduced.

## Claims

1. An expanded polytetrafluoroethylene formed article wherein a synthetic resin having a tensile elastic modulus higher than the tensile elastic modulus of polytetrafluoroethylene is applied on or impregnated into at least a part of a porous expanded polytetrafluoroethylene rod-shaped article having a porous structure consisting of fibrils and nodes connected by fibrils, and wherein the diameter of the part to which the synthetic resin is applied or impregnated is reduced by twist deformation.

2. An expanded polytetrafluoroethylene formed article according to claim 1, wherein the diameter of the part to which the synthetic resin is applied or impregnated is reduced by applying twist deformation and expansion by drawing.

3. An expanded polytetrafluoroethylene formed article according to claim 1 or claim 2, wherein the rod-shaped article is a porous expanded polytetrafluoroethylene monofilament, and wherein a synthetic resin having a tensile elastic modulus higher than the tensile elastic modulus of polytetrafluoroethylene is applied on or impregnated into one end part thereof, and wherein the diameter of the end part is reduced by applying twist deformation or by applying twist deformation and expansion by drawing.

4. A method of producing an expanded polytetrafluoroethylene formed article, comprising the steps of: (1) applying or impregnating a synthetic resin having a tensile elastic modulus higher than the tensile elastic modulus of polytetrafluoroethylene to at least a part of a porous expanded polytetrafluoroethylene rod-shaped article having a porous structure consisting of fibrils and nodes connected by fibrils; and (2) reducing, by twist deformation, the diameter of the part to which the synthetic resin has been applied or impregnated.

5. A method according to claim 4, wherein prior to the step (2), an additional step is provided such that the part to which a synthetic resin has been applied or impregnated in the step (1) is subjected to heat treatment.

6. A method according to claim 4 or claim 5, wherein after the step (2), a further step is provided such that the part whose diameter has been reduced by twist deformation is subjected to expansion by drawing.

7. A composite article comprising (a) an expanded polytetrafluoroethylene formed article in which a synthetic resin having a tensile elastic modulus higher than the tensile elastic modulus of polytetrafluoroethylene is applied on or impregnated into an end part of a porous expanded polytetrafluoroethylene rod-shaped article having a porous structure consisting of fibrils and nodes connected by fibrils and in which the diameter of the end part is reduced by a twist deformation and (b) a metal wire, wherein the diameter reduced end part of the expanded polytetrafluoroethylene formed article is inserted in a hole perforated on the cross-section of an end part of the metal wire.

8. A composite article according to claim 7, wherein the rod-shaped article is a monofilament and the metal wire is a surgical needle, and wherein the composite article comprising the monofilament suture and the surgical needle has a structure in which the end part having a reduced diameter of the monofilament is inserted in a hole perforated on the dull edge of the surgical needle.
